**Europäisches Patentamt**

**European Patent Office**

**Office européen des brevets**

(19)

(11) Veröffentlichungsnummer: **0 438 628 A2**

# (12) EUROPÄISCHE PATENTANMELDUNG

(21) Anmeldenummer: 90115008.6

(22) Anmeldetag: 04.08.90

(51) Int. Cl.⁵: **C08F 283/00**, C08F 283/06, C08F 299/02, C08F 299/06, A61K 6/08

(30) Priorität: 24.01.90 DE 4001977

(43) Veröffentlichungstag der Anmeldung:
**31.07.91 Patentblatt 91/31**

(84) Benannte Vertragsstaaten:
**AT CH DE FR GB IT LI NL SE**

(71) Anmelder: **Heraeus Kulzer GmbH**
**Heraeusstr. 12 - 14**
**W-6450 Hanau(DE)**

(72) Erfinder: **Schaefer, Roland, Dr.**
**Merianweg 5**
**W-6382 Friedrichsdorf(DE)**
Erfinder: **Eppinger, Bernhard**
**Am Kirmesplatz 17**
**W-6290 Weilburg(DE)**
Erfinder: **Mai, Gerhard, Dr.**
**Alzenauer Strasse 23**
**W-6458 Rodenbach 1(DE)**

(74) Vertreter: **Grimm, Ekkehard**
**Heraeus Holding GmbH Heraeusstrasse 12 - 14**
**W-6450 Hanau/Main(DE)**

(54) **Copolymerisierbare Mischung und ihre Verwendung.**

(57) Copolymerisierbare Mischungen aus äthylenisch ungesättigtem Monomer und darin löslichem Polybutylenglykoldi(meth)acrylat oder Polyurethandi(meth)acrylat weisen eine sehr geringe Polymerisationsschrumpfung auf und eignen sich daher besonders für die Herstellung von Formkörpern hoher Form- und Paßgenauigkeit, vorzugsweise von Zahnprothesen, Zahnfüllungen und Kronen und Brücken, und zur Verwendung in Einbettmassen für Schliffproben und Abdruckmassen für die Untersuchung von Werkstoffoberflächen.

EP 0 438 628 A2

EP 0 438 628 A2

## COPOLYMERISIERBARE MISCHUNG UND IHRE VERWENDUNG

Die Erfindung betrifft eine copolymerisierbare Mischung, die äthylenisch ungesättigtes Monomer und äthylenisch ungesättigtes Präpolymer enthält, und ihre Verwendung.

Die Herstellung von Zahnprothesen aus Kunststoff ist durch die Entwicklung des Pulver-Flüssigkeits-Verfahrens sehr vereinfacht worden. Anstelle der bis dahin benutzten thermoplastischen Kunststoffe werden seitdem plastische, gut zu verarbeitende Mischungen aus Monomeren und Polymeren eingesetzt, die infolge der mitverwendeten Polymeren während der Polymerisation wesentlich weniger schrumpfen als die reinen Monomeren. Übliche Polymere für das Pulver-Flüssigkeits-Verfahren sind das Polymethylmethacrylat und Copolymere des Methylmethacrylats; als Monomere werden meist Methacrylsäureester, vorzugsweise Methylmethacrylat, verwendet (zum Beispiel DE 737 058, DE 1 009 392, 1 544 924 und 2 530 900). Die meist in Form feiner Perlen vorliegenden Polymeren werden durch die Monomeren angelöst; die Aushärtung der Mischung erfolgt durch Polymerisation der Monomeren.

Mischungen, die durch Copolymerisation aushärten, werden in der britischen Patentschrift 1 352 063 beschrieben. Die copolymerisierbaren Mischungen bestehen aus 5 - 95 Gewichts-% Präpolymer mit Arylendioxy-, Urethan- und endständigen Vinyl-Gruppen im Molekül und aus 5 - 95 Gewichts-% mit dem Präpolymer copolymerisierbarem Vinylmonomer.

Die deutsche Offenlegungsschrift 36 32 215 betrifft einen reaktiven organischen Füllstoff in Pulverform, der Doppelbindungen enthält und zum Beispiel durch Umsetzung von Hydroxygruppen enthaltenden Acrylaten oder Methacrylaten mit Isocyanaten hergestellt werden kann. Der Füllstoff kann Vinylverbindungen als Bindemittel enthaltenden Dentalmaterialien zugesetzt werden.

Urethan-Gruppen enthaltende (Meth)Acrylsäure-Derivate, die in Dentalwerkstoffen mit wesentlicher geringerer Polymerisationsschrumpfung verwendet werden können, sind zum Beispiel aus den deutschen Offenlegungsschriften 35 22 005, 37 03 120, 37 03 130 und 37 43 782 bekannt.

Es stellt sich die Aufgabe der Erfindung, eine Mischung der eingangs charakterisierten Art mit möglichst geringer Polymerisationsschrumpfung zu finden. Die Mischung soll insbesondere auch alle die für ihre Verwendung in Dentalmaterial zur Herstellung von Zahnprothesen, künstlichen Zähnen, Kronen, Brücken, Zahnfüllungen und dergleichen erforderlichen Eigenschaften besitzen.

Die die Lösung der Aufgabe darstellende Mischung ist erfindungsgemäß dadurch gekennzeichnet, daß sie 20-90 Gewichts-% des Monomers und 10-80 Gewichts-% des Präpolymers enthält und das Präpolymer

a) ein Polybutylenglykoldiacrylat und/oder -dimethacrylat der allgemeinen Formel $RO(C_4H_8O)_pR$ mit R = Acryloyl und/oder Methacryloyl und $p \geq 35$

oder

b) ein durch Reaktion von zwei endständige Isocyanat-Gruppen aufweisendem aliphatischem Polyurethan mit 2-Hydroxyäthyl(meth)acrylat oder 2-Hydroxypropyl(meth)acrylat erhaltenes aliphatisches Polyurethandiacrylat und/oder -dimethacrylat mit einem mittleren Molekulargewicht von etwa 4000 - 6000 ist.

Besonders bewährt hat sich die Mischung, wenn sie 40 - 85 Gewichts-% des Monomers und 15 - 60 Gewichts-% des Präpolymers enthält.

Vorzugsweise besitzt das Monomer ein Molekulargewicht von höchstens 1500.

Als Monomer eignet sich jede mit dem acryliertem und/oder methacryliertem Polybutylenglykol oder Polyurethan copolymerisierbare äthylenisch ungesättigte Verbindung und ebenso ein mindestens zwei dieser Verbindungen enthaltendes Gemisch. Wird die Mischung in Dentalmaterial verwendet, kann das Monomer beziehungsweise Monomer-Gemisch aus den für diesen Zweck bekannten und üblichen Monomeren ausgewählt werden. Bevorzugt werden Ester der Acrylsäure und der Methacrylsäure mit ein- und mehrwertigen Alkoholen, besonders Acrylate und Methacrylate mit aromatischen oder cycloaliphatischen Gruppen im Alkoholrest, wie zum Beispiel das aus dem US-Patent 3 066 112 bekannte Bis-GMA (2,2-Bis-[4-(2-hydroxy-3-methacryloyloxypropoxy)-phenyl]-propan), Urethanacrylate und -methacrylate, wie das Diurethandimethacrylat aus 2,2(4),4-Trimethylhexamethylendiisocyanat und Hydroxyäthylmethacrylat, und Vinyläther, speziell 1,6-Hexandioldivinyläther (1,6-Divinyloxyhexan) und 1,4-Cyclohexandioldivinyläther (1,4-Divinyloxycyclohexan).

Die Copolymerisation der Mischung kann sowohl durch Heißpolymerisation oder Kaltpolymerisation als auch durch Photopolymerisation erfolgen.

Geeignete Katalysatoren für die Heißpolymerisation sind zum Beispiel organische Peroxide, wie Dibenzoylperoxid, für die Kaltpolymerisation zum Beispiel die Redox-Systeme, vorzugsweise solche aus organischen Peroxiden und Aminen, und für die Photopolymerisation Keton/Amin-Systeme, wie sie aus dem britischen Patent 1 408 265 bekannt sind, zum Beispiel Campherchinon/Amin.

2

Die Katalysatoren werden der Mischung in einer Menge von 0,1 - 5 Gewichts-%, vorzugsweise von 0,3 - 1 Gewichts-%, zugesetzt.

Der Mischung können, um die mechanischen Eigenschaften der daraus durch Polymerisation erhaltenen Produkte zu verbessern, feinteilige organische und/oder anorganische Füllstoffe beigefügt werden. Der Füllstoff-Anteil kann 30 - 85 Gewichts-% betragen; besonders bewährt hat sich die Mischung, wenn sie 60 - 85 Gewichts-% des Füllstoffes enthält.

Soll die Mischung für dentale Zwecke verwendet werden, so eignet sich als anorganischer Füllstoff jeder für Kunststoff-Dentalmassen übliche anorganische Füllstoff, zum Beispiel Siliciumdioxid und Silicat-Gläser, wie Lithiumaluminiumsilicat-, Strontiumaluminiumsilicat- und Bariumaluminiumsilicat-Glas.

Der besondere Vorteil der Mischung gemäß der Erfindung ist ihre sehr geringe Schrumpfung während der Polymerisation. Im Vergleich zu der theoretisch zu erwarteten Polymerisationsschrumpfung ist die bei der Polymerisation der Mischung auftretende Schrumpfung um bis zu 50 Volumen-% vermindert.

Die Mischung hat sich daher besonders bewährt, wenn die daraus durch Polymerisation hergestellten Produkte hohen Anforderungen an Form- und Paßgenauigkeit genügen müssen. Sie eignet sich deshalb besonders für den Einsatz auf dem Dentalgebiet, speziell für die Herstellung von Zahnprothesen, Zahnfüllungen und mit Kunststoff verblendeten Zahnkronen und -brücken.

Bewährt hat sich auch ihr Einsatz auf dem Gebiet der Materialprüfung, z. B. in Einbettmassen zur Herstellung von Schliffproben und in Abdruckmassen zur Untersuchung von Werkstoffoberflächen.

Zur näheren Erläuterung werden nachfolgend Beispiele für die copolymerisierbare Mischung gemäß der Erfindung und für zwei kein äthylenisch ungesättigtes Präpolymer enthaltende Mischungen (Vergleichsbeispiele 8 und 9), die Polymerisation der Mischungen und die Bestimmung der dabei auftretenden Polymerisationsschrumpfung beschrieben.

Das in den Beispielen benutzte Diurethandimethacrylat ist das Reaktionsprodukt aus 2,2(4),4-Trimethylhexamethylendiisocyanat (1 mol) und 2-Hydroxyäthylmethacrylat (2 mol).

3

Beispiel 1

|  | Gewichts-% |
|---|---|
| Diurethandimethacrylat | 60 |
| Polybutylenglykoldimethacrylat, p etwa 40 | 39 |
| Campherchinon | 0,5 |
| Dimethylaminoäthylmethacrylat | 0,5 |

Beispiel 2

|  | Gewichts-% |
|---|---|
| Diurethandimethacrylat | 17 |
| Polybutylenglykoldimethacrylat, p etwa 40 | 12 |
| Lithiumaluminiumsilicat-Glas, mittlere | |
| Teilchengröße 5 Mikrometer | 70 |
| Campherchinon | 0,5 |
| Dimethylaminoäthylmethacrylat | 0,5 |

Beispiel 3

|  | Gewichts-% |
|---|---|
| Diurethandimethacrylat | 24 |
| Polybutylenglykoldimethacrylat, p etwa 40 | 5 |
| Lithiumaluminiumsilicat-Glas, mittlere | |
| Teilchengröße 5 Mikrometer | 70 |
| Campherchinon | 0,5 |
| Dimethylaminoäthylmethacrylat | 0,5 |

Beispiel 4

|  | Gewichts-% |
|---|---|
| Diurethandimethacrylat | 60 |
| Polyurethandiacrylat aus aliphatischem Polyurethandiisocyanat und 2-Hydroxyäthylacrylat, mittleres Molekulargewicht 4900 | 39 |
| Campherchinon | 0,5 |
| Dimethylaminoäthylmethacrylat | 0,5 |

Beispiel 5

|  | Gewichts-% |
|---|---|
| Diurethandimethacrylat | 17 |
| Polyurethandiacrylat aus aliphatischem Polyurethandiisocyanat und 2-Hydroxyäthylacrylat, mittleres Molekulargewicht 4900 | 12 |
| Lithiumaluminiumsilicat-Glas, mittlere Teilchengröße 5 Mikrometer | 70 |
| Campherchinon | 0,5 |
| Dimethylaminoäthylmethacrylat | 0,5 |

Beispiel 6

|  | Gewichts-% |
|---|---|
| Hexandioldivinyläther | 50 |
| Polyurethandiacrylat aus aliphatischem Polyurethandiisocyanat und 2-Hydroxyäthylacrylat, mittleres Molekulargewicht 4900 | 49 |
| Dibenzoylperoxid | 1 |

Beispiel 7

|  | Gewichts-% |
|---|---|
| Hexandioldivinyläther | 60 |
| Polybutylenglykoldimethacrylat, p etwa 40 | 39 |
| Dibenzoylperoxid | 1 |

5

Beispiel 8     (Vergleich)

|  | Gewichts-% |
|---|---|
| Diurethandimethacrylat | 99 |
| Campherchinon | 0,5 |
| Dimethylaminoäthylmethacrylat | 0,5 |

Beispiel 9     (Vergleich)

|  | Gewichts-% |
|---|---|
| Diurethandimethacrylat | 29 |
| Lithiumaluminiumsilicat-Glas, mittlere Teilchengröße 5 Mikrometer | 70 |
| Campherchinon | 0,5 |
| Dimethylaminoäthylmethacrylat | 0,5 |

Die in den Beispielen 1 - 5 und 8 und 9 beschriebenen Mischungen werden in aus Delrin®, einem Polyacetal-Kunststoff, bestehende Röhrchen mit einer Länge von 20 mm und einem Innendurchmesser von 6,9 mm gegeben und durch 180 Sekunden langes Bestrahlen mit dem Lichtgerät Dentacolor XS der Firma Kulzer GmbH darin ausgehärtet. Die in den Beispielen 6 und 7 beschriebenen Mischungen werden in ein Edelstahl-Röhrchen mit einer Länge von 20 mm und einem Innendurchmesser von 6,9 mm gegeben und dann eine 1 Stunde lang bei 140°C ausgehärtet.

Die als lineare Schrumpfung bestimmte Polymerisationsschrumpfung ergibt sich aus der Differenz zwischen der Länge des mit der Mischung gefüllten Röhrchens $1_o$ (= 20 mm) und der Länge des ausgehärteten Formkörpers $1_l$, bezogen auf $1_o$. In der Tabelle werden die so bestimmten tatsächlichen Werte für die Polymerisationsschrumpfung, Schrumpfung$_{linear}$ [%], und - zum Vergleich dazu - die Werte für die theoretisch zu erwartende Schrumpfung angegeben.

Tabelle

| Beispiel | Schrumpfung$_{linear}$ [%] | |
| | gemessen | theoretisch |
| --- | --- | --- |
| 1 | 1,40 | 1,95 |
| 2 | 0,46 | 0,96 |
| 3 | 1,00 | 1,23 |
| 4 | 1,52 | 1,89 |
| 5 | 0,45 | 0,98 |
| 6 | 5,9 | 8,8 |
| 7 | 6,5 | 9,7 |
| 8 (Vgl.) | 2,7 | 2,9 |
| 9 (Vgl.) | 1,42 | 1,45 |

**Patentansprüche**

1. Copolymerisierbare Mischung, die äthylenisch ungesättigtes Monomer und äthylenisch ungesättigtes Präpolymer enthält, dadurch gekennzeichnet, daß sie 20 - 90 Gewichts-% des Monomers und 10 - 80 Gewichts-% eines in dem Monomer löslichen äthylenisch ungesättigten Präpolymers enthält und das Präpolymer
   a) ein Polybutylenglykoldiacrylat und/oder -dimethacrylat der allgemeinen Formel $RO(C_4H_8O)_pR$ mit R = Acryloyl und/oder Methacryloyl und $p \geqq 35$
   oder
   b) ein durch Reaktion von zwei endständige Isocyanat-Gruppen aufweisendem aliphatischem Polyurethan mit 2-Hydroxyäthyl(meth)acrylat oder 2-Hydroxypropyl(meth)acrylat erhaltenes aliphatisches Polyurethandiacrylat und/oder -dimethacrylat mit einem mittleren Molekulargewicht von etwa 4000 - 6000
   ist.

2. Mischung nach Anspruch 1, dadurch gekennzeichnet, daß sie 40 - 85 Gewichts-% des Monomers und 15 - 60 Gewichts-% des Präpolymers enthält.

3. Mischung nach Anspruch 1 oder 2, dadurch gekennzeichnet, daß das Monomer ein Molekulargewicht von höchstens 1500 besitzt.

4. Mischung nach einem der Ansprüche 1 bis 3, dadurch gekennzeichnet, daß das Monomer ein Ester der Acrylsäure und/oder Methacrylsäure ist.

5. Mischung nach einem der Ansprüche 1 bis 3, dadurch gekennzeichnet, daß das Monomer ein Vinyläther ist.

6. Mischung nach Anspruch 5, dadurch gekennzeichnet, daß das Monomer 1,6-Hexandioldivinyläther ist.

7. Mischung nach Anspruch 5, dadurch gekennzeichnet, daß das Monomer 1,4-Cyclohexandioldivinyläther ist.

8. Mischung nach einem der Ansprüche 1 bis 7, dadurch gekennzeichnet, daß sie zusätzlich 0,1 - 5 Gewichts-% eines Polymerisationskatalysators enthält.

9. Mischung nach Anspruch 8, dadurch gekennzeichnet, daß sie zusätzlich 0,3 - 1 Gewichts-% des Polymerisationskatalysators enthält.

10. Mischung nach Anspruch 8 oder 9, dadurch gekennzeichnet, daß der Katalysator ein Katalysator für die Photopolymerisation ist.

11. Mischung nach einem der Ansprüche 1 bis 10, dadurch gekennzeichnet, daß sie 30 bis 80 Gewichts-% eines feinteiligen anorganischen Füllstoffes enthält.

12. Verwendung der Mischung nach einem der Ansprüche 1 bis 11 in Dentalmaterialien.

13. Verwendung der Mischung nach einem der Ansprüche 1 bis 11 in Einbettmassen für Schliffproben.

14. Verwendung der Mischung nach einem der Ansprüche 1 bis 11 in Abdruckmassen für die Untersuchung von Werkstoffoberflächen.